# EUROPEAN PATENT SPECIFICATION

(11) **EP 2 863 838 B1**
(45) Date of publication and mention of the grant of the patent: **11.09.2019**
(21) Application number: 13807263.2
(22) Date of filing: 21.06.2013
(51) Int. Cl.: A61F 2/02, A61L 27/24, A61L 27/14, A61L 27/38, A61C 8/02

(54) **COMPRESSION AND KINK RESISTANT IMPLANTS**
KOMPRESSIONS- UND KNICKBESTÄNDIGE IMPLANTATE
IMPLANTS RÉSISTANT À LA COMPRESSION ET AU PLIAGE

(30) Priority: 22.06.2012 US 201213530322
(43) Date of publication of application: 29.04.2015
(73) Proprietor: Collagen Matrix, Inc., Oakland, NJ 07436 (US)
(72) Inventor: LI, Shu-Tung, Wyckoff, NJ 07481 (US); YUEN, Debbie, Woodcliff Lake, NJ 07675 (US)
(74) Representative: Chajmowicz, Marion
(86) International application number: PCT/US2013/046950
(87) International publication number: WO 2013/192481

(56) References cited:
- WO-A1-2004/087231
- WO-A1-2010/117766
- WO-A2-03/011149
- WO-A2-2005/046457
- US-A1- 2009 024 150
- US-A1- 2011 276 066
- US-A1- 2011 288 628
- US-B2- 8 133 500
- Glycolate ET AL: "Novel Absorbable Monomers and Polymers for Biomedical Applications INTRODUCTION", , 31 December 2009 (2009-12-31), XP055235816, Retrieved from the Internet: URL:http://www.bezwadabiomedical.com/files /publications/2009/1-NAMP.pdf [retrieved on 2015-12-11]

## Description

### BACKGROUND

The major clinical objective in the repair of a severed nerve is to restore continuity between the proximal and distal nerve stumps, without which functional recovery is virtually impossible. Typically, when the distal and proximal nerve stumps can be brought into continuity without much tension, direct suture or re-coaptation repair is the preferred treatment. In cases where there is a nerve gap distance that must be bridged, some type of intervening material must be used. The most commonly used material is an autograft of a peripheral nerve harvested from the patient, such as a sural nerve autograft. However, the results of nerve autografting are typically not satisfactory. Axonal escape at the suture lines reduces the number of axons reaching the end organ. It also can lead to painful neuroma formation. Further, harvesting of an autograft necessitates a second surgery and its associated complications. Additional problems of nerve autograft include failure of graft survival and vascularization and size mismatch.

Alternative nerve graft products that can improve the shortcomings of a nerve autograft have been developed. Such products include nerve guide tubes or conduits for guiding peripheral nerve regeneration, so called "entubulation repair."

A multi-layered, semipermeable nerve guide conduit that promotes *in vivo* nerve regeneration is described in U.S. Patent 4,963,146. Since the nerve guide conduit is made as a straight tube, it does not provide kink resistance that is important for repairing nerves in areas that require bending of the implant for proper connection (such as nerves in the wrist and hand). Kinking of the nerve guide tube can cause nerve compression, axonal disruption, and neuroma formation.

A kink resistant nerve repair implant is described in U.S. Patent 6,716,225. In this implant, ridges were created along the wall of the nerve guide to impart kink resistance to it. However, the ridges in the wall, upon hydration, tend to relax, causing the total length of the nerve guide to increase by as much as 30% as compared to its length in the dry state. As such, the extent of kink resistance will be reduced and the effectiveness of the implant in areas requiring a high degree of kink resistance will be minimized. Additionally, the ridges do not prevent the implant from collapsing in vivo. Thus, external forces from surrounding tissues can compress the implant wall and reduce the luminal space required for axonal growth. As a result, the effectiveness of the nerve guiding mechanism is significantly compromised. Also, this implant is not effective for repair of longer gaps, e.g., longer than 2.5 cm. US2011/288628 discloses implantable tubular prosthesis comprising a support structure wrapped helically around the tubular body to increase kink and crush resistance, WO2010/117766 discloses resorbable collagen membranes for augmenting alveolar bone.

In order to correct the deficiencies of current nerve guides and improve peripheral nerve repair of long gaps, there is a need to develop a resorbable nerve guide that is both compression resistant and kink resistant during the period of nerve regeneration so as to avoid significant mechanical distortion of the implant lumen. Such an implant can also be used to repair other tubular organs, e.g., tendon, vascular tissue, and urological tissue. A need also exists for a compression-resistant implant for use in areas requiring maintenance of space for tissue growth, e.g., ridge augmentation in dental surgeries.

### SUMMARY

The main objective of this invention is to provide implants for tissue repair and regeneration, particularly for nerve repair and for ridge augmentation in dental surgery, which eliminate or reduce the disadvantages and problems associated with currently available implants.

Thus, one aspect of this invention relates to a compression and kink resistant implant for nerve repair. The implant includes a tubular biopolymeric membrane and a polymeric filament according to claim 1. The tubular biopolymeric membrane is biocompatible, resorbable, and semipermeable. The polymeric filament is generally helical and is located on the outer surface of the tubular biopolymeric membrane. The implant is compression and kink resistant. The implant has a compression resistance of 1 N to 10 N and a kink resistance angle of 40 degrees to 150 degrees.

Another aspect of this invention relates to a shaped compression resistant implant for ridge augmentation in dental surgery. The shaped implant contains an arcuate biopolymeric membrane that includes a polymeric filament on its surface according to claim 9. The arcuate biopolymeric membrane is biocompatible, resorbable, and semipermeable. The shaped implant is compression resistant and has a compression resistance of 1 N to 10 N. In an alternative embodiment, the shaped compression resistant implant can have two arcuate biopolymeric layers that are biocompatible, resorbable, and semipermeable. A polymeric filament is incorporated between the two layers of the arcuate biopolymeric membrane. The two-layered implant can have a compression resistance of 1 N to 10 N.

Also provided is a method for preparing a compression and kink resistant tubular implant according to claim 13. The method includes the steps of dispersing purified biopolymeric fibers, hydrating the dispersed purified collagen fibers to form reconstituted collagen fibers, winding the reconstituted collagen fibers onto a rotating mandrel to form a collagen tube, winding a synthetic polymer filament onto the surface of the collagen tube, partially dehydrating the collagen tube, freeze drying the partially dehydrated collagen tube, and crosslinking the freeze-dried partially dehydrated collagen tube to form the compression and kink resistant tubular implant.

Additionally provided is a method for preparing a compression resistant implant. The method includes steps in which purified collagen fibers are dispersed, the dispersed purified collagen fibers are hydrated to form reconstituted collagen fibers, a first portion of the reconstituted collagen fibers are wound onto a rotating mandrel to form a collagen tube, a synthetic polymer filament is wound onto the surface of the collagen tube, a second portion of the reconstituted collagen fibers is wound onto the surface of the collagen tube to form a collagen layer encasing the synthetic polymer filament and the collagen tube, the encased collagen tube is partially dehydrated, freeze dried, and cut along a longitudinal axis to form a sheet. The sheet thus formed is humidified, molded into an arcuate shape, and crosslinked to form the compression resistant implant. In an alternative embodiment, the step of winding a second portion of collagen fibers around the collagen tube is omitted. This method forms a compression resistant implant having a single collagen layer with a synthetic polymer filament on its surface.

The details of one or more embodiments of the invention are set forth in the accompanying drawings and the description below. Other features, objects, and advantages of the invention will be apparent from the description and drawing, and from the claims.

### BRIEF DESCRIPTION OF THE DRAWINGS

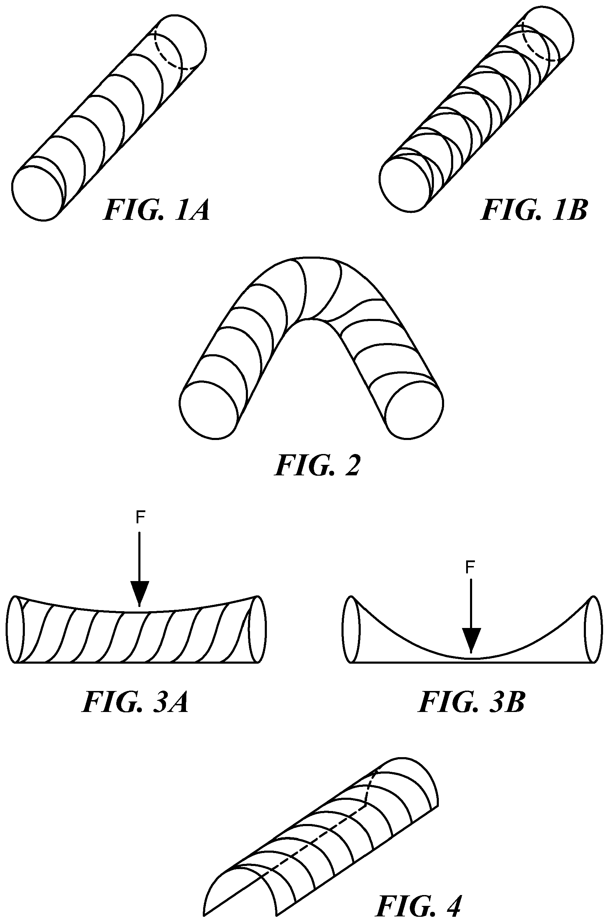

FIG. 1A is a schematic representation of a compression-resistant and kink-resistant implant in which a polymer filament is helically wrapped around a tubular matrix. FIG. 1B depicts an alternative embodiment of an implant having a crisscross wrapping of the filament.
FIG. 2 shows bending of the implant depicted in FIG. 1A, demonstrating the kink resistant aspect of the invention.
FIG. 3A shows the superior compression resistance of the implant depicted in FIG. 1A as compared to FIG. 3B that shows a non-reinforced tubular matrix having low compression resistance.
FIG. 4 shows a compression-resistant implant for dental ridge augmentation.
FIG. 5 is a plot of compression resistance versus time for polymer fiber-reinforced or control nerve guide implants.
FIG. 6 is a plot of number of myelinated axons versus implant luminal area.

### DETAILED DESCRIPTION

This invention relates to a biocompatible, resorbable, semipermeable, compression-resistant and kink-resistant tubular biopolymeric matrix implant circumferentially supported by a synthetic polymeric filament wound around the surface of its outer wall.

The tubular biopolymeric matrix implant of the present invention is biocompatible, resorbable, and semipermeable. That is, the tubular implant is slowly resorbed in vivo by endogenous enzymes. The tubular biopolymeric matrix may be manufactured from biological materials including, but not limited to collagen, elastin, polysaccharides such as alginic acid, chitosan, and cellulose, and from genetically engineered biological materials. Collagen-based materials are preferred, particularly type I collagen-based materials. The implant can have an internal diameter of 1.0 mm to 10 mm, preferably from 1.5 mm to 8 mm, and more preferably from 1.5 mm to 6 mm. The length of the implant can be 0.5 cm to 15 cm, preferably from 1.0 cm to 10 cm, and more preferably from 1.5 cm to 8 cm. For example, a tubular biopolymeric matrix implant for nerve repair can have an internal diameter of 1.5 mm to 6.0 mm.

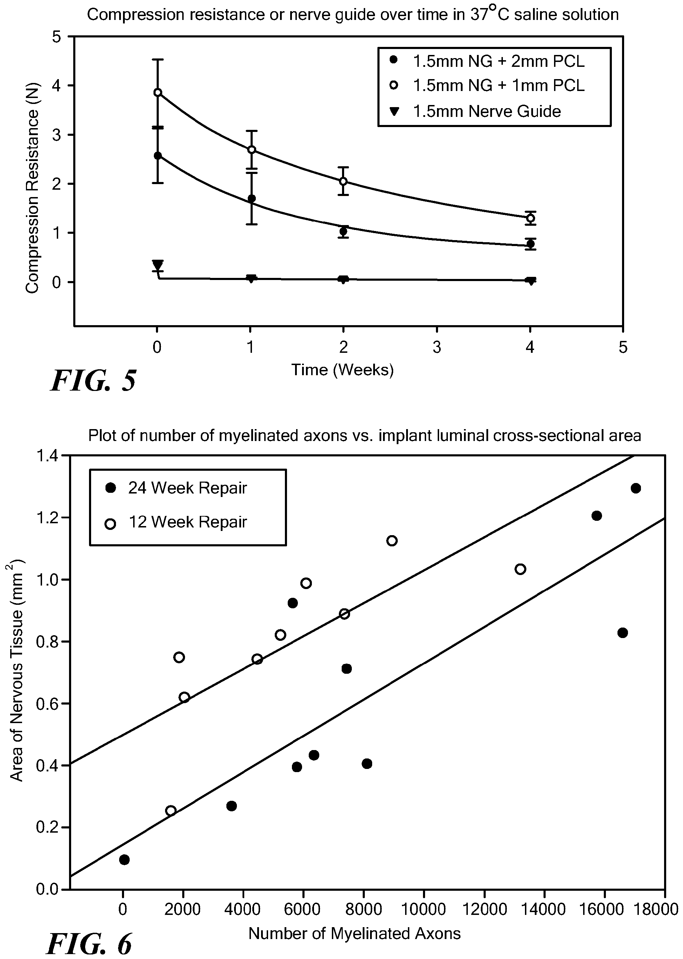

The implant is compression resistant. This property is imparted by a polymeric filament that is wound around the outside of the tubular biopolymeric matrix in a helical path. The extent of compression resistance is a function of the pitch of the filament winding. For example, an implant having a polymeric filament wound with a small pitch, i.e., a tight winding, has a higher compression resistance as compared to a similar implant having a winding with a larger pitch. The implant has a winding density such that its compression resistance is between 1 N and 10N. The winding density is preferably selected to impart a compression resistance of 2 N to 5 N. The relationship between polymeric filament pitch and compression resistance is shown in FIG. 5. For example, an implant having an inside diameter of 1.5 mm that is reinforced with a polymeric fiber wound with a 1 mm pitch has a compression resistance of 4 N. A similar implant in which the polymeric fiber is wound with a 2 mm pitch has a compression resistance of 2.5 N. In an alternative embodiment, the polymeric fiber is wound in a crisscross pattern with a small diameter filament such that the thickness of the implant wall is not significantly increased. The compression resistance imparted by a crisscross polymeric fiber is greater than that of a helical fiber given the same winding pitch.

The implant is also kink resistant. The kink resistance, similar to the compression resistance described above, is accomplished by helical or crisscross winding of the polymer filament over the tubular collagen matrix. The degree of kink resistance is defined as the angle at which the implant kinks. A kink is defined as a sharp bend which causes an occlusion of the lumen of the tubular implant. The implant has a kink resistance angle from about 40 degrees to about 150 degrees, preferably from about 50 degrees to about 90 degrees.

The polymer supported implant will, in use, advantageously maintain its overall length, a significant improvement over the implant described in U.S. Patent 6,716,225.

The polymeric filament is biodegradable and is constructed of synthetic polymers selected from polyglicolic acid, polylactic acid, copolymers of polyglicolic acid and polylactic acid, polycaprolactone, copolymers of polylactic acid and polycaprolactone, and copolymers of polyglicolic acid and polycaprolactone. The polymeric filament is biodegraded via hydrolysis of the polymer.

The polymeric filament may be incorporated on the surface of the tubular wall or may be incorporated inside the wall space. When the filament is wound outside the wall the diameter of the filament can be larger than if the filament is wound inside the wall space.

Depending on the length of the nerve to be repaired, the rate of degradation of the implant can be programmed to fulfill the functional need of the implant *in vivo.* For example in the case of nerve repair, an axon grows at a rate of approximately 1 mm per day. To repair a nerve defect of 3-5 cm, the implant should have an in vivo stability of about 2-4 months. The control of the *in vivo* stability can be accomplished by using chemical crosslinking agents that form intermolecular covalent bonds between the biopolymeric molecules. Crosslinking can be carried out by means well known in the art such as those described in US Patent 6,090,996. In brief, crosslinking can be conducted in a chamber with a relative humidity in the range from 80% to 100% in the presence of an excess amount of formaldehyde vapor at a temperature of 25°C for a period of 1 hour to 10 hours. For example, crosslinking can be accomplished by exposing the tubular biopolymeric matrix to a 0.5% formaldehyde solution for 5 hours at room temperature.

The *in vivo* stability can also be controlled by selecting the appropriate polymer filament that compliment the resorption characteristic of the tubular collagen matrix implant.

The implant can contain a micro-guiding system to facilitate cell adhesion and migration, such as that described in US Patent 6,716,225.

The implant can also contain bioactive molecules to either promote axon growth or cell adhesion and migration. Bioactive molecules for promoting axon growth include nerve growth factors, acidic and basic fibroblast growth factors, and insulin-like growth factors. These growth factors promote mitogenesis of cells within the implant lumen such as Schwann cells or stem cells.

Bioactive molecules for promoting cell adhesion and migration include bioadhesive molecules such as laminins, fibronectins, glycoproteins, and glycosaminoglycans. The bioactive molecules can be incorporated into the wall of the nerve implant or can be incorporated via a delivery vehicle that can be inserted into the lumen of the implant. The growth factors and adhesive molecules may be incorporated into the implant via electrostatic interactions, physical and mechanical interactions, covalent interactions using a crosslinking agent, or via a delivery matrix (e.g., porous collagen sponge) that are well known in the art.

Cells that have a therapeutic indication can be incorporated into the implant. These cells include, but are not limited to, Schwann cells and stem cells.

Another property of the implant is selective permeability. The implant is permeable to molecules of up to 500,000 daltons. Preferably, the implant is permeable to molecules of 5,000 to 100,000 daltons. Most bioactive molecules and nutrient molecules have a molecular weight in this range.

In another embodiment, a shaped compression resistant implant according to claim 9 is provided. This implant is especially useful for surgical applications in which space for bone growth must be maintained, such as for dental ridge augmentation surgery. A tubular compression and kink resistant implant having the properties described above is cut along a longitudinal direction to form a sheet. The polymer fiber-reinforced sheet membrane is then mechanically shaped over a mold and crosslinked to fix its shape. The shaped membrane will maintain compression resistance for the particular medical or dental surgical application.

The specific examples below are to be construed as merely illustrative, and not limitative of the remainder of the disclosure in any way whatsoever. Without further elaboration, it is believed that one skilled in the art can, based on the description herein, utilize the present invention to its fullest extent.

### EXAMPLE 1: Preparation of a tubular compression and kink resistant nerve repair implant Preparation of insoluble collagen fibers

Bovine flexor tendon was cleaned by removing fat and fascia and by washing with water. The cleaned tendon was frozen and comminuted into 0.5 mm slices with a meat slicer. One kilogram of the sliced wet tendon was subsequently extracted with 5 L of distilled water, followed by 5 L of 0.2 N HCl/0.5 M Na₂SO₄ at room temperature for 24 hours. The extraction solution was discarded.

The residual acid in the extracted tendon was removed by washing with 5 L of a 0.5M Na₂SO₄ solution. The tendon was then extracted with 5 L of a 1.0 M NaOH/0.75 M Na₂SO₄ solution at room temperature for 24 hours. The extraction solution again was discarded. Any residual base was neutralized by adding a 0.1N HCl solution to achieve a pH of 5, followed by several washes with distilled water to remove residual salts in the purified tendon. The tendon was then defatted for 8 hours with 5 volumes of isopropanol at room temperature under constant agitation, followed by an overnight treatment with an equal volume of isopropanol. The resulting insoluble collagen fiber preparation was then air-dried and stored at room temperature until further processing.

### Preparation of a collagen fiber dispersion

An aliquot of the insoluble collagen fibers was weighed and dispersed in 0.07 M lactic acid, homogenized with a Silverson Homogenizer (East Longmeadow, MA), and filtered with a 30 mesh stainless steel mesh filter to obtain a dispersion containing 0.7% (w/v) collagen. The dispersion was de-aerated under vacuum to remove the air trapped in the dispersion and stored at 4°C until use.

### Preparation of a polymer fiber-reinforced tubular collagen matrix

An aliquot of acid dispersed collagen fibers prepared as described above was reconstituted by adding 0.3% NH₄OH to adjust the pH of the dispersion to the isoelectric point of collagen, i.e., pH 4.5-5.0. The reconstituted fibers were poured into a fabrication device which was set up with the insertion of a mandrel of 1.5 mm in diameter. The fibers were evenly distributed along the mandrel. The mandrel was then slowly rotated at about 40-50 rpm to firmly wind the fibers around it, thus forming a tubular collagen matrix.

A polylactide-polycaprolactone (PCL) copolymer filament was slowly wound with a pitch of 2 mm onto the surface of the tubular collagen matrix. The collagen fibers in the tubular collagen matrix were partially dehydrated by removing excess solution by compressing the tubular collagen matrix on the rotating mandrel against two plates to precisely control the thickness of the wall of the tubular collagen matrix.

### Freeze-drying and cross-linking of the polymer fiber-reinforced tubular collagen matrix

The partially dehydrated collagen fibers in the polymer fiber-reinforced tubular collagen matrix were freeze-dried at -10°C for 24 hours and at 20°C for 16 hours under a pressure less than 200 millitorr using a Virtis Freeze Dryer (Gardiner, NY). The freeze-dried polymer fiber-reinforced tubular matrix was removed from the mandrel and cross-linked with formaldehyde vapor generated from a 3% formaldehyde solution at ambient temperature for about 7 hours. The crosslinked polymer fiber-reinforced tubular matrix was rinsed in water to remove residual formaldehyde and freeze-dried again.

### EXAMPLE 2: Preparation of a comparative tubular implant

A comparative tubular implant was prepared as described above in Example 1 for the tubular compression and kink resistant nerve repair implant, except that the PCL polymer filament was omitted.

### EXAMPLE 3: Preparation of a shaped compression and kink resistant implant

A polymer fiber-reinforced tubular collagen matrix was prepared as described in Example 1 above except that the mandrel used had a diameter of 10 mm. Following freeze-drying of the polymer fiber-reinforced tubular collagen matrix, the tube is cut longitudinally and removed from the mandrel. The resulting sheet of polymer-reinforced collagen matrix was then humidified in a closed chamber having a relative humidity from 90% to 100% at room temperature for 2 to 4 hours. Following humidification, the sheet was pressed onto a mold to form it into an arch shape. The matrix sheet was then cross-linked while being held in the arch shape, rinsed, and freeze-dried again as described in Example 1.

### EXAMPLE 4: Preparation of an alternative embodiment of a shaped compression and kink resistant implant

A polymer fiber-reinforced tubular collagen matrix was prepared as described in Example 1 above. A second layer of reconstituted collagen fibers were then evenly distributed along the polymer fiber-reinforced tubular collagen matrix. The mandrel was then slowly rotated at about 40-50 rpm to firmly wind the fibers around it, thus forming a second layer of collagen matrix.

The collagen fibers in the two layers of tubular collagen matrix were partially dehydrated by removing excess solution by compressing the tubular collagen matrix on the rotating mandrel against two plates to precisely control the thickness of the wall of the tubular collagen matrix.

The resulting double-layer tube was then freeze-dried and cut longitudinally to remove it from the mandrel. The resulting sheet of polymer-reinforced double-layer collagen matrix was then humidified as described in Example 3 above, and then pressed onto a mold to form the sheet into an arch shape. The double-layer matrix sheet was then cross-linked while being held in the arch shape, rinsed, and freeze-dried again as described in Example 1.

### EXAMPLE 5: Characterization of the implants

### Permeability

Tubular implants having an inside diameter (ID) of 1.5mm and a length of 5-6 cm were first hydrated in 0.01M phosphate buffer, pH 7.0, and then filled with 50 µl of a 5mg/ml solution containing a probe molecule. Probe molecules included glucose (MW 180 Dal), myoglobin (MW 16,000 Dal), carbonic anhydrase (MW 29,000 Dal), bovine serum albumin (BSA: MW 67,000 Dal), β-galactosidase (MW 456,000 Dal), and blue dextran (MW 2x10⁶ Dal). After clamping closed the ends of the nerve repair implants, they were placed in a chamber containing 10 ml of 0.01M phosphate buffer, pH 7.0, and allowed to equilibrate for 24 hours at room temperature. Probe molecules which permeated through the nerve implant membrane were measured by the Bradford assay for proteins and the anthrone assay for carbohydrates.

Permeability of a shaped implant was measured in a two-compartment chamber in which the shaped implant separates the two chambers. The probe molecules were introduced into one of the chambers and allowed to diffuse across the shaped implant for 24 hours. Then the amount of probe molecules in the other chamber was measured after 24 hours as described above.

### Density

Tubular implants were dried in a desiccator over P₂O₅ for 24 hours and their dry weight determined using an analytical balance (Mettler model AE240). The length, ID, and outside diameter (OD) were then measured using a caliper (Mitutoyo). Density was calculated as dry weight divided by volume [(π r²_{OD} L) - (π r²_{ID} L)], where r_{OD} = radius of the OD, r_{ID} = radius of the ID, and L = length of the implant. For non-tubular samples, the thickness, area, and weight of the implant were measured and the density was then calculated accordingly.

### Kink Resistance

Tubular implants were hydrated in distilled water for 5 minutes. The implants were aligned along the bottom edge of a protractor and both ends of the implant were bent to form an angle. The degree of kink resistance was defined as the angle at which the implant kinks. A kink is defined as a sharp bend which causes an occlusion of the lumen of the tubular implant.

### Suture Pull-out Strength

Tubular implants were cut open along their length and hydrated in water for 5 minutes. A 3-0 silk suture was placed approximately 3 mm from the edge of the tube along the longitudinal orientation and attached to a mechanical platform test stand (Chatillon TCD-200, Greensboro, NC). The sample was slowly pulled apart at a rate of 2.54 cm/min and the tension at which the suture pulled out was measured by a Chatillon DFGS2 digital force gauge.

### Compression Resistance

Tubular implants were hydrated in water for 5 minutes. Samples were placed onto a Chatillon TCD-200 test stand. The samples were slowly compressed at a rate of 1.27 cm/min until the walls of the tube came into contact with each other. The compression force required was measured by a Chatillon DFGS2 digital force gauge.

Compression resistance of a shaped implant was measured in a similar manner, except that the sample was compressed until the implant wall came into contact with the base of the test stand.

### Hydrothermal Transition Temperature (Tₛ)

Hydrothermal transition temperatures were measured using a differential scanning calorimeter (Mettler/Toledo DSC882). A sample was punched out from an implant and placed in a 40 µl aluminum pan with 20 µl of 0.01M phosphate-buffered saline, pH 7.0 and sealed. The Tₛ was measured at a heating rate of 5°C/min and taken as peak readings.

### Table 1 below summarizes the results of in vitro characterization of tubular implants.

**Table 1. Characterization of Tubular Implants***

| Characteristics | Comparative (no polymer reinforcement) | Polymer fiber reinforced |
|---|---|---|
| Wall Thickness (mm) | 0.41 ± 0.02 [4] | 0.41 ± 0.02 [4] |
| Kink Resistance (degrees) | 46 ± 5 [4] | 80 ± 4 [4] |
| Suture Pull-out strength (kg) | 0.13 ± 0.031 [4] | 0.25 ± 0.059 [4] |
| Compression Resistance (N) | 0.19 ± 0.04 [4] | 3.4 ± 0.43 [4] |
| Hydrothermal Transition Temperature (°C) | 61 ± 2.1 [4] | 64 ± 0.7 [3] |

| Permeability (%) | | |
|---|---|---|
| Myoglobin (MW 16,000) | 81 ± 9.3 [6] | 67 ± 7.9 [6] |
| Carbonic Anhydrase (MW 29,000) | 53 ± 19 [6] | 41 ± 8.0 [6] |
| BSA (MW 67,000) | 36 ± 10 [6] | 22 ± 6 [6] |
| β-galactosidase (MW 456,000) | 24 ± 5 [6] | 16 ± 3 [6] |

| | | |
|---|---|---|
| *Data reported as mean ± standard deviation. Number in [] indicates number of samples tested. | | |

The results of the characterization studies showed that the polymer fiber-reinforced tubular implant is both compression and kink resistant. The implant membrane is permeable to molecules up to the size of BSA, a size comparable to many nutrient molecules and growth factors. The hydrothermal transition temperature indicated that the implant has an *in vivo* resorption time of about 6-12 months based on previous studies. See Yuen, D., Ulreich, J.B., Zuclich, G., Lin, H.B. and Li, S.T., 2000, "Prediction of in vivo stability of a resorbable, reconstituted type I collagen membrane by in vitro methods" Trans. Sixth World Biomaterials Congress, p. 222.

Additionally, as shown in FIG. 5, the polymer fiber-reinforced tubular implant remains kink resistant even following incubation in saline at 37°C for 4 weeks.

### EXAMPLE 6: Animal Studies

The rat sciatic nerve was used as a model to evaluate nerve repair implants. Female Lewis rats (250-300g) were anesthetized with sodium pentobarbital, followed by shaving and cleaning of the incision site prior to exposing the sciatic nerve.

In a control autograft group, a 10 mm section of the sciatic nerve was excised, inverted, rotated 180°, and sutured back into place with 10-0 nylon suture. In a second control group and the experimental group, a 5 mm segment of the nerve was excised, resulting in a 10 mm gap after retraction of the transected nerve. Two millimeters of each nerve stump was inserted into each end of a 14 mm tubular implant lacking a polymer fiber reinforcement (second control group) or into a 14 mm tubular compression and kink resistant nerve repair implant of the instant invention (experimental group) and sutured in place with 10-0 nylon suture, resulting in a gap of 10 mm. The repair of the sciatic nerve was followed for 12 and 24 weeks.

Histological and histomorphometrical analyses were conducted using the cross sectional view of light micrographs at the mid-section of the regenerated nerve.

In the experimental group, all tubular compression and kink resistant nerve repair implants maintained their circular cross sectional area with minimal geometrical distortion. Nerve regeneration was robust following 12 weeks of surgery. At this time point, most of the implants' lumen space had filled with regenerated axons, and the collagen fibers, although partially degraded, still maintained their intact appearance.

At 24 weeks post-surgery, the lumen was completely filled with regenerated axons. The regenerated nerve core was round and, in most of the specimens, the original implant had completely degraded and resorbed. In some of the specimens, the margin between the nerve core tissue and the implant could not be identified.

In the control group that received the implant lacking a polymer fiber reinforcement, nerve regeneration was also quite robust at both time points. Due to the low compression resistance of the control nerve implant, some nerve implant's cross sections showed an elongated shape. Additionally, the overall size and shape of the regenerated nerve in cross section at both 12 and 24 weeks varied between animals, reflecting a variation in the degree of shrinkage of the individual implant. Most of the collagen fibers of the control implants were resorbed at 12 weeks post-surgery.

In the autograft control group, nerve regeneration at both 12 and 24 weeks was robust. The regeneration appeared largely within the epineural sheath domain of the autograft. However, the overall size and shape of the regenerated nerve in cross section at both time points varied from animal to animal, reflecting an intrinsic variability in the size of the autograft.

Table 2 below summarizes the results of the histomorphometrical studies described above. In all repair groups, an increase in the number of myelinated axons was observed from week 12 to week 24. Use of the inventive implant, as compared to the control non-reinforced implant, unexpectedly resulted in a greater number of myelinated axons at both time points. Animals that received the inventive implant had a number of myelinated axons similar to animals in the autograft group at 12 weeks and greater than the autograft group animals at 24 weeks. When compared to the autograft group, the inventive implant had the most similar results in terms of number of myelinated axons, the size of myelinated axons, the area occupied by the regenerated nerve, and the area occupied by the myelinated axons. This finding indicates that nerve regeneration using the inventive implant is comparable to that obtained using an autograft, the gold standard for nerve repair and regeneration.

**TABLE 2. Summary of Histomorphometric Analysis**

| Time (weeks) | Type of Repair | Total Number of Myelinated Axons | Average Axon Diameter (µm) | Nerve Tissue Area (mm²) | % Area Occupied by Axons |
|---|---|---|---|---|---|
| 12 | Non-reinforced | 3567 ± 717 [10] | 3.61 ± 0.47 [10] | 0.58 ± 0.108 [10] | 12.54 ± 3.257 [10] |
| | Present invention | 5556 ± 1254 [9] | 3.95 ± 0.54 [9] | 0.80 ± 0.09 [9] | 7.96 ± 1.084 [9] |
| | Autograft | 5424 ± 1203 [8] | 3.56 ± 0.56 [8] | 1.25 ± 0.280 [8] | 6.38 ± 1.558 [8] |
| | Normal | 5598 ± 480 [8] | 9.18 ± 1.17 [8] | 0.62 ± 0.032 [8] | 55.65 ± 9.005 [8] |
| 24 | Non-reinforced | 5413 ± 1441 [8] | 3.99 ± 0.67 [7] | 0.53 ± 0.131 [8] | 14.18 ± 3.183 [8] |
| | Present invention | 8621 ± 1849 [10] | 4.20 ± 0.58 [10] | 0.65 ± 0.127 [10] | 17.60 ± 2.763 [10] |
| | Autograft | 5692 ± 590 [8] | 4.59 ± 0.76 [8] | 1.25 ± 0.28 [8] | 10.27 ± 2.170 [8] |
| | Normal | 6298 ± 171 [9] | 9.40 ± 1.21 [8] | 0.62 ± 0.03 [9] | 71.4 ± 5.57 [9] |

| | | | | | |
|---|---|---|---|---|---|
| *Data reported as mean ± standard error of the mean Number in [ ] represents the number of animals included in the data analysis | | | | | |

Nerve regeneration facilitated by implantation of the present invention was characterized by a linear correlation between the number of myelinated axons versus implant luminal cross-sectional area. As shown in FIG. 6, the correlation coefficient of a plot of these two parameters measured at 12 and 24 weeks was 0.61 and 0.71, respectively. This finding was consistent with the axonal distribution within the luminal space. The correlation coefficient increased with increasing time of implantation, indicating that myelinated axons were more evenly distributed in the luminal space at 24 weeks as compared to 12 weeks. The non-reinforced control implant did not show such a correlation.

This finding confirms the ability of the tubular compression and kink resistant nerve repair implant to advantageously maintain its structural integrity throughout the entire regeneration process of the peripheral nerve. Currently available commercial collagen-based nerve repair products are recommended for the repair of short gaps, i.e. <2.5cm. The physical and physico-chemical characteristics of the compression and kink resistance nerve implant of the present invention, taken together with the results of the animal study presented above indicates that the present implant can be used to bridge nerve gaps longer than 2.5cm in humans.

## Claims

1. A compression and kink resistant implant for nerve repair, comprising a tubular biopolymeric membrane manufactured of collagen-based materials and a biodegradable synthetic polymeric filament, the tubular biopolymeric membrane having an outer surface and being biocompatible, resorbable, and semipermeable, the biodegradable synthetic polymeric filament being helical and located on the outer surface of the tubular biopolymeric membrane, wherein the implant has a compression resistance of 1 N to 10 N and a kink resistance angle of 40 degrees to 150 degrees, and wherein the biodegradable synthetic polymeric filament is formed of a polymer selected from the group consisting of polyglicolic acid, polylactic acid, copolymers of polyglicolic acid and polylactic acid, polycaprolactone, copolymers of polylactic acid and polycaprolactone, and copolymers of polyglicolic acid and polycaprolactone.

2. The implant of claim 1, wherein the tubular biopolymeric membrane includes type I collagen.

3. The implant of claim 1, wherein the implant has an internal diameter of 1.0 mm to 10 mm.

4. The implant of claim 1, wherein the implant has a length of 0.5 cm to 15 cm.

5. The implant of claim 1, wherein the tubular biopolymeric membrane has a thickness of 0.1 mm to 1 mm.

6. The implant of claim 1, wherein the biodegradable synthetic polymeric filament has a helical pitch of 1 mm to 2 mm.

7. The implant of claim 1, wherein the biodegradable synthetic polymeric filament is present in a crisscross arrangement.

8. The implant of claim 1, wherein the tubular biopolymeric membrane is permeable to molecules having a molecular weight < 500,000 daltons, preferably < 100,000 daltons.

9. A shaped compression resistant implant for ridge augmentation in dental surgery, comprising an arcuate biopolymeric membrane manufactured of collagen-based materials and a biodegradable synthetic polymeric filament, the arcuate biopolymeric membrane having an outer surface and being biocompatible, resorbable, and semipermeable, and the biodegradable synthetic polymeric filament being located on the outer surface of the arcuate biopolymeric membrane, wherein the implant has a compression resistance of 1 N to 10 N, and wherein the biodegradable synthetic polymeric filament is formed of a polymer selected from the group consisting of polyglicolic acid, polylactic acid, copolymers of polyglicolic acid and polylactic acid, polycaprolactone, copolymers of polylactic acid and polycaprolactone, and copolymers of polyglicolic acid and polycaprolactone.

10. The implant of claim 9, wherein the arcuate biopolymeric membrane includes type I collagen.

11. The implant of claim 9, further comprising a second arcuate biopolymeric membrane, wherein the biodegradable synthetic polymeric filament is incorporated between the arcuate biopolymeric membrane and the second arcuate biopolymeric membrane, and wherein the implant has a compression resistance of 1 N to 10 N.

12. The implant of claim 11, wherein the second arcuate biopolymeric membrane includes type I collagen.

13. A method for preparing a compression and kink resistant tubular implant, comprising dispersing purified collagen fibers, coacervating the dispersed purified collagen fibers to form reconstituted collagen fibers, winding the reconstituted collagen fibers onto a rotating mandrel to form a collagen tube, winding a biodegradable synthetic polymer filament onto the surface of the collagen tube, partially dehydrating the collagen tube, freeze drying the partially dehydrated collagen tube, and crosslinking the freeze-dried partially dehydrated collagen tube to form a compression and kink resistant tubular implant, wherein the biodegradable synthetic polymer filament is preferably wound in a criss-cross pattern, and wherein the biodegradable synthetic polymeric filament is formed of a polymer selected from the group consisting of polyglicolic acid, polylactic acid, copolymers of polyglicolic acid and polylactic acid, polycaprolactone, copolymers of polylactic acid and polycaprolactone, and copolymers of polyglicolic acid and polycaprolactone.

14. A method for preparing a compression resistant implant, comprising dispersing purified collagen fibers, coacervating the dispersed purified collagen fibers to form reconstituted collagen fibers, winding a first portion of the reconstituted collagen fibers onto a rotating mandrel to form a collagen tube, winding a biodegradable synthetic polymer filament onto the surface of the collagen tube, winding a second portion of the reconstituted collagen fibers onto the surface of the collagen tube to form a collagen layer encasing the biodegradable synthetic polymer filament and the collagen tube, partially dehydrating the encased collagen tube, freeze drying the partially dehydrated encased collagen tube, cutting the freeze-dried encased collagen tube along a longitudinal axis to form a sheet, humidifying the sheet, molding it into an arcuate shape, and crosslinking the molded sheet to form a compression resistant implant, wherein the biodegradable synthetic polymer filament is preferably wound in a criss-cross pattern, and wherein the biodegradable synthetic polymeric filament is formed of a polymer selected from the group consisting of polyglicolic acid, polylactic acid, copolymers of polyglicolic acid and polylactic acid, polycaprolactone, copolymers of polylactic acid and polycaprolactone, and copolymers of polyglicolic acid and polycaprolactone.

## Patentansprüche

1. Ein kompressions- und knickbeständiges Implantat zur Nervenreparatur, umfassend eine röhrenförmige Biopolymermembran, hergestellt aus Kollagen-basierten Materialien, und ein bioabbaubares synthetisches polymeres Filament, wobei die röhrenförmige Biopolymermembran eine Außenseite besitzt und biokompatibel, resorbierbar und semipermeabel ist, wobei das bioabbaubare synthetische polymere Filament spiralförmig ist und sich auf der Außenseite der röhrenförmigen Biopolymermembran befindet, wobei das Implantat einen Kompressionswiderstand von 1 N bis 10 N und einen Knickwiderstandswinkel von 40 Grad bis 150 Grad besitzt und wobei das bioabbaubare synthetische polymere Filament aus einem Polymer gebildet ist, ausgewählt aus der Gruppe, bestehend aus Polyglykolsäure, Polymilchsäure, Copolymeren von Polyglykolsäure und Polymilchsäure, Polycaprolacton, Copolymeren von Polymilchsäure und Polycaprolacton und Copolymeren von Polyglykolsäure und Polycaprolacton.

2. Das Implantat nach Anspruch 1, wobei die röhrenförmige Biopolymermembran Kollagen Typ I umfasst.

3. Das Implantat nach Anspruch 1, wobei das Implantat einen Innendurchmesser von 1,0 mm bis 10 mm besitzt.

4. Das Implantat nach Anspruch 1, wobei das Implantat eine Länge von 0,5 cm bis 15 cm besitzt.

5. Das Implantat nach Anspruch 1, wobei die röhrenförmige Biopolymermembran eine Dicke von 0,1 mm bis 1 mm besitzt.

6. Das Implantat nach Anspruch 1, wobei das bioabbaubare synthetische polymere Filament eine Spiralsteigung von 1 mm bis 2 mm besitzt.

7. Das Implantat nach Anspruch 1, wobei das bioabbaubare synthetische polymere Filament in einer kreuzgewickelten Anordnung vorliegt.

8. Das Implantat nach Anspruch 1, wobei die röhrenförmige Biopolymermembran für Moleküle mit einem Molekulargewicht < 500.000 Dalton, vorzugsweise < 100.000 Dalton permeabel ist.

9. Ein geformtes kompressionsbeständiges Implantat zur Kammaugmentation in der Dentalchirurgie, umfassend eine bogenförmige Biopolymermembran, hergestellt aus Kollagen-basierten Materialien, und ein bioabbaubares synthetisches polymeres Filament, wobei die bogenförmige Biopolymermembran eine Außenseite besitzt und biokompatibel, resorbierbar und semipermeabel ist und das bioabbaubare synthetische polymere Filament sich auf der Außenseite der bogenförmigen Biopolymermembran befindet, wobei das Implantat einen Kompressionswiderstand von 1 N bis 10 N besitzt und wobei das bioabbaubare synthetische polymere Filament aus einem Polymer gebildet ist, ausgewählt aus der Gruppe, bestehend aus Polyglykolsäure, Polymilchsäure, Copolymeren von Polyglykolsäure und Polymilchsäure, Polycaprolacton, Copolymeren von Polymilchsäure und Polycaprolacton und Copolymeren von Polyglykolsäure und Polycaprolacton.

10. Das Implantat nach Anspruch 9, wobei die bogenförmige Biopolymermembran Kollagen Typ I umfasst.

11. Das Implantat nach Anspruch 9, außerdem umfassend eine zweite bogenförmige Biopolymermembran, wobei das bioabbaubare synthetische polymere Filament zwischen der bogenförmigen Biopolymermembran und der zweiten bogenförmigen Biopolymermembran eingebaut ist und wobei das Implantat eine Kompressionswiderstand von 1 N bis 10 N besitzt.

12. Das Implantat nach Anspruch 11, wobei die zweite bogenförmige Biopolymermembran Kollagen Typ I umfasst.

13. Ein Verfahren zur Herstellung eines kompressions- und knickbeständigen röhrenförmigen Implantats, umfassend Dispergieren gereinigter Kollagenfasern, Koazervieren der dispergierten gereinigten Kollagenfasern, um rekonstituierte Kollagenfasern zu bilden, Wickeln der rekonstituierten Kollagenfasern auf einer Drehspindel, um eine Kollagenröhre zu bilden, Wickeln eines bioabbaubaren synthetischen polymeren Filaments auf der Oberfläche der Kollagenröhre, partielles Dehydrieren der Kollagenröhre, Gefriertrocknen der partiell dehydrierten Kollagenröhre und Vernetzen der gefriergetrockneten partiell dehydrierten Kollagenröhre, um ein kompressions- und knickbeständiges röhrenförmiges Implantat zu bilden, wobei das bioabbaubare synthetische polymere Filament vorzugsweise in einem kreuzgewickelten Muster gewickelt ist und wobei das bioabbaubare synthetische polymere Filament aus einem Polymer gebildet ist, ausgewählt aus der Gruppe, bestehend aus Polyglykolsäure, Polymilchsäure, Copolymeren von Polyglykolsäure und Polymilchsäure, Polycaprolacton, Copolymeren von Polymilchsäure und Polycaprolacton und Copolymeren von Polyglykolsäure und Polycaprolacton.

14. Ein Verfahren zur Herstellung eines kompressionsbeständigen Implantats, umfassend Dispergieren gereinigter Kollagenfasern, Koazervieren der dispergierten gereinigten Kollagenfasern, um rekonstituierte Kollagenfasern zu bilden, Wickeln eines ersten Teils der rekonstituierten Kollagenfasern auf einer Drehspindel, um eine Kollagenröhre zu bilden, Wickeln eines bioabbaubaren synthetischen polymeren Filaments auf der Oberfläche der Kollagenröhre, Wickeln eines zweiten Teils der rekonstituierten Kollagenfasern auf der Oberfläche der Kollagenröhre, um eine Kollagenschicht zu bilden, die das bioabbaubare synthetische polymere Filament und die Kollagenröhre ummantelt, partielles Dehydrieren der ummantelten Kollagenröhre, Gefriertrocknen der partiell dehydrierten ummantelten Kollagenröhre, Schneiden der gefriergetrockneten ummantelten Kollagenröhre entlang einer Längsachse, um eine Schicht zu bilden, Befeuchten der Schicht, Formen dieser zu einer Bogenform und Vernetzen der geformten Schicht, um ein kompressionsbeständiges Implantat zu bilden, wobei das bioabbaubare synthetische polymere Filament vorzugsweise in einem kreuzgewickelten Muster gewickelt ist und wobei das bioabbaubare synthetische polymere Filament aus einem Polymer gebildet ist, ausgewählt aus der Gruppe, bestehend aus Polyglykolsäure, Polymilchsäure, Copolymeren von Polyglykolsäure und Polymilchsäure, Polycaprolacton, Copolymeren von Polymilchsäure und Polycaprolacton und Copolymeren von Polyglykolsäure und Polycaprolacton.

## Revendications

1. Implant résistant à la compression et à la torsion pour réparation d'un nerf, comprenant une membrane biopolymère tubulaire fabriquée avec des matériaux à base de collagène et un filament polymère synthétique biodégradable, la membrane biopolymère tubulaire ayant une surface extérieure et étant biocompatible, résorbable, et semi-perméable, le filament polymère synthétique biodégradable étant hélicoïdal et situé sur la surface extérieure de la membrane biopolymère tubulaire, lequel implant a une résistance à la compression de 1 N à 10 N et un angle de résistance à la torsion de 40 degrés à 150 degrés, dans lequel le filament polymère synthétique biodégradable est formé d'un polymère choisi dans le groupe constitué par le poly(acide glycolique), le poly(acide lactique), les copolymères de poly(acide glycolique) et de poly(acide lactique), la polycaprolactone, les copolymères de poly(acide lactique) et de polycaprolactone, et les copolymères de poly(acide glycolique) et de polycaprolactone.

2. Implant selon la revendication 1, dans lequel la membrane biopolymère tubulaire comprend du collagène de type I.

3. Implant selon la revendication 1, lequel implant a un diamètre interne de 1,0 mm à 10 mm.

4. Implant selon la revendication 1, lequel implant a une longueur de 0,5 cm à 15 cm.

5. Implant selon la revendication 1, dans lequel la membrane biopolymère tubulaire a une épaisseur de 0,1 mm à 1 mm.

6. Implant selon la revendication 1, dans lequel le filament polymère synthétique biodégradable a un pas d'hélice de 1 mm à 2 mm.

7. Implant selon la revendication 1, dans lequel le filament polymère synthétique biodégradable est présent selon un agencement croisé.

8. Implant selon la revendication 1, dans lequel la membrane biopolymère tubulaire est perméable à des molécules ayant un poids moléculaire < 500 000 daltons, de préférence < 100 000 daltons.

9. Implant résistant à la compression façonné pour reconstitution de la crête alvéolaire en chirurgie dentaire, comprenant une membrane biopolymère arquée fabriquée avec des matériaux à base de collagène et un filament polymère synthétique biodégradable, la membrane biopolymère arquée ayant une surface extérieure et étant biocompatible, résorbable, et semi-perméable, et le filament polymère synthétique biodégradable étant situé sur la surface extérieure de la membrane biopolymère arquée, lequel implant a une résistance à la compression de 1 N à 10 N, dans lequel le filament polymère synthétique biodégradable est formé d'un polymère choisi dans le groupe constitué par le poly(acide glycolique), le poly(acide lactique), les copolymères de poly(acide glycolique) et de poly(acide lactique), la polycaprolactone, les copolymères de poly(acide lactique) et de polycaprolactone, et les copolymères de poly(acide glycolique) et de polycaprolactone.

10. Implant selon la revendication 9, dans lequel la membrane biopolymère arquée comprend du collagène de type I.

11. Implant selon la revendication 9, comprenant en outre une deuxième membrane biopolymère arquée, dans lequel le filament polymère synthétique biodégradable est incorporé entre la membrane biopolymère arquée et la deuxième membrane biopolymère arquée, et dans lequel l'implant a une résistance à la compression de 1 N à 10 N.

12. Implant selon la revendication 11, dans lequel la deuxième membrane biopolymère arquée comprend du collagène de type I.

13. Méthode de préparation d'un implant tubulaire résistant à la compression et à la torsion, comprenant la dispersion de fibres de collagène purifiées, la coacervation des fibres de collagène purifiées dispersées pour former des fibres de collagène reconstituées, l'enroulement des fibres de collagène reconstituées sur un mandrin rotatif pour former un tube de collagène, l'enroulement d'un filament polymère synthétique biodégradable sur la surface du tube de collagène, la déshydratation partielle du tube de collagène, la lyophilisation du tube de collagène partiellement déshydraté, et la réticulation du tube de collagène partiellement déshydraté lyophilisé pour former un implant tubulaire résistant à la compression et à la torsion, dans laquelle le filament polymère synthétique biodégradable est de préférence enroulé selon un motif croisé, et dans laquelle le filament polymère synthétique biodégradable est formé d'un polymère choisi dans le groupe constitué par le poly(acide glycolique), le poly(acide lactique), les copolymères de poly(acide glycolique) et de poly(acide lactique), la polycaprolactone, les copolymères de poly(acide lactique) et de polycaprolactone, et les copolymères de poly(acide glycolique) et de polycaprolactone.

14. Méthode de préparation d'un implant résistant à la compression, comprenant la dispersion de fibres de collagène purifiées, la coacervation des fibres de collagène purifiées dispersées pour former des fibres de collagène reconstituées, l'enroulement d'une première partie des fibres de collagène reconstituées sur un mandrin rotatif pour former un tube de collagène, l'enroulement d'un filament polymère synthétique biodégradable sur la surface du tube de collagène, l'enroulement d'une deuxième partie des fibres de collagène reconstituées sur la surface du tube de collagène pour former une couche de collagène enveloppant le filament polymère synthétique biodégradable et le tube de collagène, la déshydratation partielle du tube de collagène enveloppé, la lyophilisation du tube de collagène enveloppé partiellement déshydraté, la découpe du tube de collagène enveloppé lyophilisé le long d'un axe longitudinale pour former une feuille, l'humidification de la feuille, son moulage sous une forme arquée, et la réticulation de la feuille moulée pour former un implant résistant à la compression, dans laquelle le filament polymère synthétique biodégradable est de préférence enroulé selon un motif croisé, et dans laquelle le filament polymère synthétique biodégradable est formé d'un polymère choisi dans le groupe constitué par le poly(acide glycolique), le poly(acide lactique), les copolymères de poly(acide glycolique) et de poly(acide lactique), la polycaprolactone, les copolymères de poly(acide lactique) et de polycaprolactone, et les copolymères de poly(acide glycolique) et de polycaprolactone.
